(19) Europäisches Patentamt
European Patent Office
Office européen des brevets

(11) Veröffentlichungsnummer: **0 432 135 A1**

(12) **EUROPÄISCHE PATENTANMELDUNG**

(21) Anmeldenummer: **91101254.0**

(51) Int. Cl.[5]: **C07C 205/37**, C07C 201/12

(22) Anmeldetag: **23.02.87**

Diese Anmeldung ist am 31 - 01 - 1991 als Teilanmeldung zu der unter INID-Kode 60 erwähnten Anmeldung eingereicht worden.

(30) Priorität: **28.02.86 CH 808/86**
**19.11.86 CH 4617/86**
**24.12.86 CH 5238/86**

(43) Veröffentlichungstag der Anmeldung:
**12.06.91 Patentblatt 91/24**

(60) Veröffentlichungsnummer der früheren Anmeldung nach Art. 76 EPÜ: **0 235 089**

(84) Benannte Vertragsstaaten:
**AT BE CH DE ES FR GB GR IT LI NL**

(71) Anmelder: **CIBA-GEIGY AG**
**Klybeckstrasse 141**
**CH-4002 Basel(CH)**

(72) Erfinder: **Drabek, Jozef, Dr.**
**Benkenstrasse 12**
**CH-4104 Oberwil(CH)**

(54) **Zwischenprodukte für Benzoylphenylharnstoffe.**

(57) Neue substituierte Nitrobenzole der Formel I

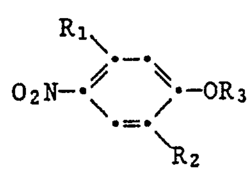

(I),

worin $R_1$ und $R_2$ je Halogen bedeuten, wobei $R_1$ und $R_2$ nicht gleichzeitig Chlor sind und $R_3$ für $-CF_2-CHF-CF_3$ steht und Verfahren zu ihrer Herstellung, werden offenbart. Die Zwischenprodukte dienen der Herstellung von insektiziden N-Benzoyl-N'-2,5-dihalogen-4-hexafluorpropyloxyphenyl-harnstoffen, deren bevorzugtes Anwendungsgebiet die Bekämpfung von Schädlingen an Tieren und Pflanzen ist.

EP 0 432 135 A1

## ZWISCHENPRODUKTE FÜR BENZOYLPHENYLHARNSTOFFE

Die vorliegende Erfindung betrifft neue substituierte Nitrobenzole als Ausgangsmaterialien für insektizid wirksame N-Benzoyl-N'-2,5-dihalogen-4-halogenalkoxyphenylharnstoffe und Verfahren zu ihrer Herstellung.

Die erfindungsgemässen Verbindungen entsprechen der Formel I

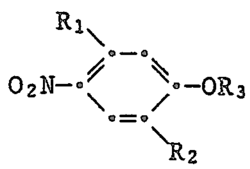

(I)

worin $R_1$ und $R_2$ je Halogen bedeuten, wobei $R_1$ und $R_2$ nicht gleichzeitig Chlor sind und $R_3$ für $-CF_2-CHF-CH_3$ steht.

Die Verbindungen der Formel I sind wertvolle Zwischenprodukte für die Herstellung von insektizid wirksamen Benzoylharnstoffen der Formel

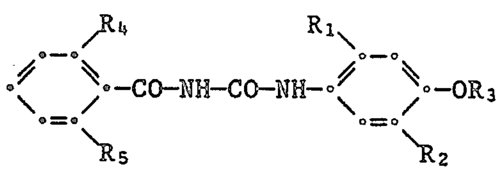

worin $R_1$ und $R_2$ je Halogen bedeuten, wobei $R_1$ und $R_2$ nicht gleichzeitig Chlor sind, $R_3$ $-CF_2-CHF-CF_3$, $R_4$ für Wasserstoff, Halogen, Methyl, Methoxy oder Methylthio und $R_5$ für Halogen, Methyl, Methoxy oder Methylthio stehen. Diese insektiziden Wirkstoffe sind Gegenstand der Europäischen Patentanmeldung EP-A-235089.

Bei den als Substituenten in Betracht kommenden Halogenen handelt es sich sowohl um Fluor und Chlor als auch um Brom und Jod, wobei Fluor, Chlor und Brom bevorzugt sind.

Unter den Verbindungen der Formel I stehen diejenigen im Vordergrund, in denen $R_1$ und $R_2$ je Fluor, Chlor oder Brom bedeuten, wobei $R_1$ und $R_2$ nicht gleichzeitig Chlor sind und $R_3$ für $-CF_2-CHF-CF_3$ steht.

Dabei sind diejenigen Verbindungen der Formel I bevorzugt, in denen $R_1$ Fluor und $R_2$ Fluor, Chlor oder Brom oder $R_1$ Chlor und $R_2$ Fluor bedeuten und $R_3$ für $-CF_2-CHF-CF_3$ steht.

Die Nitroverbindungen der Formel I sind z.B. herstellbar durch Haloalkylierung eines 2,5-Dihalogen-4-nitrophenols (vgl. französische Patentschrift 2,005,876) oder durch Umsetzen von 2,5-Dihalogen-4-fluornitrobenzol mit einem Halogenalkanol in alkalischer Lösung und Dimethylsulfoxid als Lösungsmittel (vgl. "The Chemistry of the Hydroxyl group", Seiten 83-124; Interscience Publishers Inc., New York, 1971).

Zur weiteren Umsetzung zu den insektizid wirksamen Benzoylharnstoffen der EP-A-235089, werden die analogen Aniline der Formel II

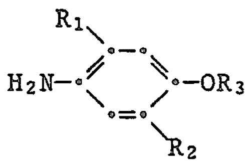

(II)

benötigt. Die erhält man, indem man die Nitrobenzole der Formel I in Analogie zu dem in J. Org. Chem. 29 (1964), 1, aufgezeigten Verfahren hydriert (vgl. auch die dort zitierte Literatur). Aber auch durch chemische Reduktion (z.B. mittels Sn-(II)-Chlorid/HCl) der erfindungsgemässen Nitroverbindungen der Formel I sind Aniline der Formel II zugänglich (Vgl. Houben Weyl, "Methoden d. org. Chemie" 11/1, 422). Daraus erhält man die insektizid wirksamen Benzoylharnstoffe indem man entweder

a) ein Anilin der Formel II

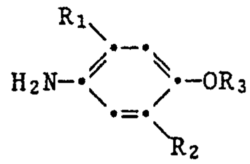

(II)

mit einem Benzoylisocyanat der Formel III

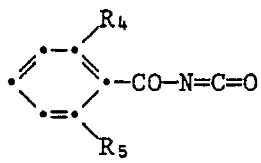

(III),

oder

b) ein Isocyanat der Formel IV

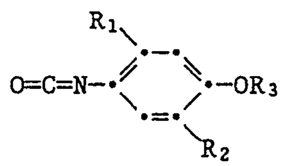

(IV)

mit einem Benzamid der Formel V

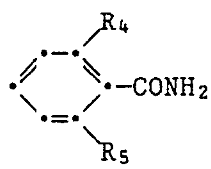

(V),

oder

c) ein Anilin der Formel II mit einem Urethan der Formel VI

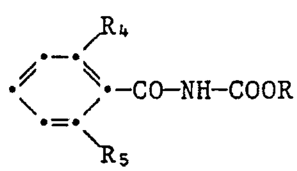

(VI)

umsetzt. In den obigen Formeln II bis VI haben die Reste $R_1$, $R_2$, $R_3$, $R_4$ und $R_5$ die für Formel I angegebene Bedeutung und R steht für einen $C_1$-$C_8$-Alkylrest, der gegebenenfalls mit Halogen, vorzugsweise Chlor, substituiert sein kann.

Die Ausgangsstoffe der Formeln III und V sind bekannt und können analog bekannten Verfahren hergestellt werden.

Die neuen Ausgangsstoffe der Formel II bilden ebenfalls einen Gegenstand der EP-A-235089.

Die 4-Haloalkoxy-phenylisocyanate der Formel IV lassen sich z.B. durch Phosgenierung der Aniline der Formel II nach allgemein üblichen Verfahren herstellen. Die weiterhin als Ausgangsstoffe zu verwendenden

Benzamide der Formel V sind bekannt (vgl. z.B. Beilstein "Handbuch der organischen Chemie" Bd. 9, S. 336).

Urethane der Formel VI können in an sich bekannter Weise erhalten werden durch Umsetzung eines Benzoylisocyanats der Formel III mit einem entsprechenden Alkohol oder durch Umsetzung eines Benzamides der Formel V in Anwesenheit einer basischen Verbindung mit einem entsprechenden Ester der Chlorameisensäure Cl-COOR.

Beispiel: Herstellung

Nitrobenzole

2-Fluor-4-(2,2,2-trifluoräthoxy)-5-chlor-nitrobenzol

22,5 g 88%iges Kaliumhydroxid werden in 120 ml Dimethylsulfoxid suspendiert. Zu dieser Suspension werden tropfenweise unter Rühren 40 g Trifluoräthanol gegeben. Die entstandene Lösung lässt man unter Rühren bei Raumtemperatur in eine Lösung von 96,8 g 2,4-Difluor-5-chlor-nitrobenzol in 150 ml Dimethylsulfoxid tropfen. Nach beendeter Zugabe wird noch 2 Stunden bei Raumtemperatur weitergerührt. Alsdann wird das Reaktionsgemisch eingeengt, das Rohprodukt in Methylenchlorid gelöst, die Lösung mit Wasser gewaschen und getrocknet und schliesslich das Lösungsmittel abdestilliert. Die Reinigung erfolgt chromatographisch an einer Kieselgelsäule mit Hexan/Aether 10:1 als Laufmittel. Nach Abdestillation des Lösungsmittels liegt die Titelverbindung der Formel

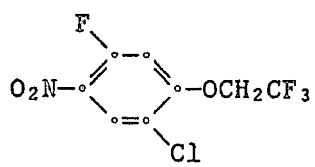

in Form gelber Kristalle vor; Smp. 54-55 ° C.

Aniline

2-Fluor-4-(2,2,2-trifluoräthoxy)-5-chloranilin

21,3 g des obigen Nitrobenzols werden in 220 ml Tetrahydrofuran gelöst und bei Raumtemperatur während 19 Stunden in Gegenwart von 4 g Raney-Nickel hydriert ($H_2$-Aufnahme: 5,26 1). Das Reaktionsgemisch wird filtriert, das Lösungsmittel abdestilliert und der Rückstand destilliert. Die Titelverbindung der Formel

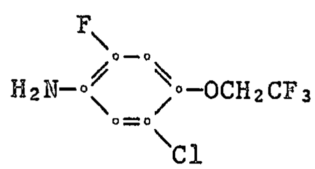

weist einen Siedepunkt von 166 ° C/0,08 Torr und einen Schmelzpunkt von 40-41 ° C auf.

2-Fluor-(4-1,1,2,3,3,3-hexafluorpropoxy)-5-bromanilin

28 g 2-Brom-4-amino-5-fluorphenol und 1,9 ml Triäthylamin werden in 150 ml Dimethylformamid vorgelegt worauf 22,5 g Hexafluorpropylen bei Raumtemperatur eingeleitet werden. Dabei steigt die Temperatur auf 50 ° C. Anschliessend wird das Lösungsmittel im Vakuum abdestilliert, der Rückstand in Aether aufgenommen und die ätherische Lösung zweimal mit verdünnter Natronlauge und zweimal mit Wasser extrahiert. Der über Natriumsulfat getrocknete ätherische Auszug wird fraktioniert destilliert. Die Titelverbindung der Formel

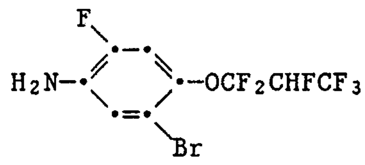

liegt in Form einer hellgelben Flüssigkeit vor; Sdp. 76-81 °C/ 0,001 Torr.

Auf analoge Weise werden die folgenden Verbindungen hergestellt:

| $R_1$ | $R_2$ | $R_3$ | phys. Daten |
|---|---|---|---|
| F | Cl | $CF_2CHFCF_3$ | Sdp. 74-76°C/0,01 Torr |
| F | F | $CF_2CHFCF_3$ | Sdp. 56-58°C/0,15 Torr |

Endprodukt

N-(2,6-Difluorbenzoyl)-N'-[2-fluor-4-(2,2,2-trifluoräthoxy)-5-chlor-phenyl]-harnstoff

4 g 2-Fluor-4-(2,2,2-trifluoräthoxy)-5-chlor-anilin, gelöst in 50 ml trockenem Toluol, werden bei Raumtemperatur mit 3,0 g 2,6-Difluorbenzoylisocyanat, gelöst in 10 ml trockenem Toluol, versetzt und 10 Stunden lang gerührt. Anschliessend werden etwa 75 % des Lösungsmittels am Rotationsverdampfer entfernt. Der gebildete Niederschlag wird abgenutscht, mit wenig kaltem Toluol und Hexan gewaschen und im Vakuum getrocknet. Die Titelverbindung der Formel

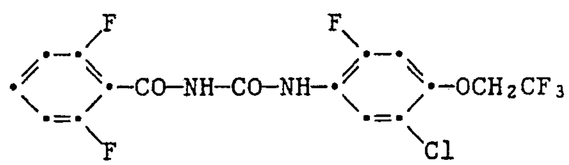

liegt als weisses, kristallines Pulver vor; Smp. 201-202 °C.

Auf analoge Weise werden die folgenden Verbindungen hergestellt:

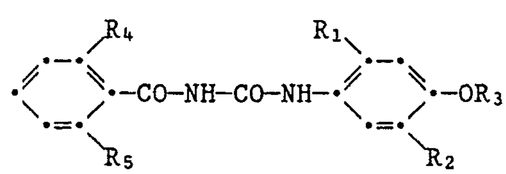

| $R_1$ | $R_2$ | $R_3$ | $R_4$ | $R_5$ | Smp. °C |
|---|---|---|---|---|---|
| F | Br | $CF_2CHFCF_3$ | F | F | 159-161 |
| F | Br | $CF_2CHFCF_3$ | H | Cl | 142-143 |
| F | Br | $CF_2CHFCF_3$ | H | F | 139-140 |
| F | Br | $CF_2CHFCF_3$ | F | Cl | 190-192 |
| F | Br | $CF_2CHFCF_3$ | F | $OCH_3$ | 172-174 |
| F | F | $CF_2CHFCF_3$ | F | F | 165-166 |
| F | F | $CF_2CHFCF_3$ | H | Cl | 169-170 |
| F | F | $CF_2CHFCF_3$ | H | F | 163-164 |
| F | F | $CF_2CHFCF_3$ | F | Cl | 191-193 |
| F | Cl | $CF_2CHFCF_3$ | H | F | 135-136 |
| F | Cl | $CF_2CHFCF_3$ | F | Cl | 198-199 |
| F | Cl | $CF_2CHFCF_3$ | Cl | Cl | 184-185 |
| F | Cl | $CF_2CHFCF_3$ | F | $OCH_3$ | 172-173 |
| F | Cl | $CF_2CHFCF_3$ | H | $OCH_3$ | 162-163,5 |
| F | Cl | $CF_2CHFCF_3$ | $OCH_3$ | $OCH_3$ | 175-177 |
| F | Br | $CF_2CHFCF_3$ | Cl | Cl | 181-182 |
| F | Br | $CF_2CHFCF_3$ | H | $OCH_3$ | 159-161 |
| F | Br | $CF_2CHFCF_3$ | $OCH_3$ | $OCH_3$ | 184-185 |
| F | F | $CF_2CHFCF_3$ | Cl | Cl | |
| F | F | $CF_2CHFCF_3$ | F | $OCH_3$ | |
| F | F | $CF_2CHFCF_3$ | H | $OCH_3$ | |
| F | F | $CF_2CHFCF_3$ | $OCH_3$ | $OCH_3$ | |

## Ansprüche

1. Verbindungen der Formel I

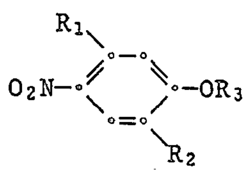

(I),

worin $R_1$ und $R_2$ je Halogen bedeuten, wobei $R_1$ und $R_2$ nicht gleichzeitig Chlor sind, und $R_3$ für $-CF_2-CHF-CF_3$ steht.

2. Verbindungen der Formel I gemäss Anspruch 1, worin $R_1$ und $R_2$ je Fluor, Chlor oder Brom bedeuten, wobei $R_1$ und $R_2$ nicht gleichzeitig Chlor sind, und $R_3$ für $-CF_2-CHF-CF_3$ steht.

3. Verbindungen der Formel I gemäss Anspruch 2, worin $R_1$ Fluor und $R_2$ Fluor, Chlor oder Brom oder $R_1$ Chlor und $R_2$ Fluor bedeuten, und $R_3$ für $-CF_2-CHF-CF_3$ steht.

4. Verbindungen der Formel I gemäss Anspruch 3, worin $R_1$ Fluor, $R_2$ Fluor, Chlor oder Brom und $R_3$ für den Rest $-CF_2-CHF-CF_3$ steht.

5. Verfahren zur Herstellung der Verbindungen der Formel I gemäss Anspruch 1, dadurch gekennzeichnet, dass man ein 4-Nitrophenol der Formel

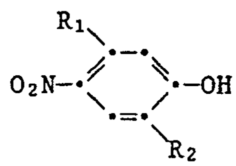

worin $R_1$ und $R_2$ die unter Formel I gegebene Bedeutung haben, mit Hexafluorpropylen umsetzt.

Patentansprüche: (für den Vertragsstaat Spanien)

1. Verfahren zur Herstellung der Verbindungen der Formel I

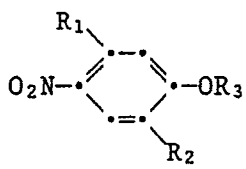

(I),

worin $R_1$ und $R_2$ je Halogen bedeuten, wobei $R_1$ und $R_2$ nicht gleichzeitig Chlor sind, und $R_3$ für $-CF_2-CHF-CF_3$ steht, dadurch gekennzeichnet, dass man ein 4-Nitrophenol der Formel

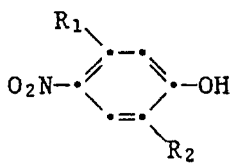

worin $R_1$ und $R_2$ die unter Formel I gegebene Bedeutung haben, mit Hexafluorpropylen umsetzt.

2. Verfahren gemäss Anspruch 1, worin $R_1$ und $R_2$ je Fluor, Chlor oder Brom bedeuten, wobei $R_1$ und $R_2$ nicht gleichzeitig Chlor sind, und $R_3$ für $-CF_2-CHF-CF_3$ steht.

3. Verfahren gemäss Anspruch 2, worin $R_1$ Fluor und $R_2$ Fluor, Chlor oder Brom oder $R_1$ Chlor und $R_2$ Fluor bedeuten, und $R_3$ für $-CF_2-CHF-CF_3$ steht.

4. Verfahren gemäss Anspruch 3, worin $R_1$ Fluor, $R_2$ Fluor, Chlor oder Brom und $R_3$ für den Rest $-CF_2-CHF-CF_3$ steht.

Europäisches Patentamt

# EUROPÄISCHER RECHERCHENBERICHT

Nummer der Anmeldung

**EP 91 10 1254**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | FR-A-2 005 876 (FARBWERKE HOECHST AG VORMALS MEISTER LUCINS & BRÜNING)<br>* Patentanspruch 1 *<br>- - - | 5 | C 07 C 205/37<br>C 07 C 201/12 |
| A | PATENT ABSTRACTS OF JAPAN, Band 7, Nr. 171 (C-178)[1316], 28. Juli 1983;<br>& JP-A-58 79 960 (SUMITOMO KAGAKU KOGYO K.K.) 13-05-1983<br>* Zusammenfassung *<br>- - - - - | 1 | |

| RECHERCHIERTE SACHGEBIETE (Int. Cl.5) |
|---|
| C 07 C 205/00<br>C 07 C 201/00 |

Der vorliegende Recherchenbericht wurde für alle Patentansprüche erstellt

| Recherchenort | Abschlußdatum der Recherche | Prüfer |
|---|---|---|
| Den Haag | 12 März 91 | BONNEVALLE E.I.H. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument